(19)

Europäisches
Patentamt
European
Patent Office
Office européen
des brevets

(11) **EP 3 763 285 A1**

(12) **EUROPEAN PATENT APPLICATION**
published in accordance with Art. 153(4) EPC

(43) Date of publication:
**13.01.2021 Bulletin 2021/02**

(51) Int Cl.:
***A61B 5/026*** (2006.01)        ***A61B 6/00*** (2006.01)

(21) Application number: **18908596.2**

(22) Date of filing: **13.06.2018**

(86) International application number:
**PCT/CN2018/091166**

(87) International publication number:
**WO 2019/169779 (12.09.2019 Gazette 2019/37)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **05.03.2018 CN 201810178339**

(71) Applicant: **Suzhou Rainmed Medical Technology
Co., Ltd.
Jiangsu 215123 (CN)**

(72) Inventors:
• HUO, Yunfei
  **Suzhou, Jiangsu 215123 (CN)**
• LIU, Guangzhi
  **Suzhou, Jiangsu 215123 (CN)**

• WANG, Zhiyuan
  **Suzhou, Jiangsu 215123 (CN)**
• ZHANG, Hailing
  **Suzhou, Jiangsu 215123 (CN)**
• HUO, Yong
  **Suzhou, Jiangsu 215123 (CN)**
• GONG, Yanjun
  **Suzhou, Jiangsu 215123 (CN)**
• LI, Jianping
  **Suzhou, Jiangsu 215123 (CN)**
• YI, Tieci
  **Suzhou, Jiangsu 215123 (CN)**
• YANG, Fan
  **Suzhou, Jiangsu 215123 (CN)**

(74) Representative: **Gerauer, Marc Philippé
Kraus & Weisert
Patentanwälte PartGmbB
Thomas-Wimmer-Ring 15
DE-80539 München (DE)**

(54) **METHOD FOR MEASURING FRACTIONAL FLOW RESERVE WITHOUT VASODILATOR**

(57)    Provided is a method for measuring a fractional flow reserve without a vasodilator, including the following steps: measuring a pressure P1 at a coronary artery inlet by a blood pressure sensor; acquiring a two-dimensional vessel diameter and length of a blood vessel by an angiography image, and generating a three-dimensional blood vessel grid model and acquiring a three-dimensional vessel diameter and length of the blood vessel; measuring a time taken for blood flowing from a start point to an end point of a segment of the blood vessel and calculating a blood flow velocity V1 based on the time and the three-dimensional length of the blood vessel; calculating an aortic pressure P2 in a maximum hyperemia state; calculating a blood flow velocity V2 in the maximum hyperemia state; taking the blood flow velocity V2 as a velocity at the coronary artery inlet and the aortic pressure P2 as a pressure Pa at the coronary artery inlet, calculating a pressure drop ΔP from the coronary artery inlet to a distal end of a coronary stenosis, a mean intracoronary pressure Pd at the distal end of the stenosis Pd = Pa-ΔP; and, by the formula CAFFR = Pd/Pa, and calculating coronary angiography fractional flow reserve CAFFR based on the formula of CAFFR=Pd/Pa. The advantage of the disclosure is that the fractional flow reserve can be calculated by an angiography image, an aortic pressure and blood flow, without using the vasodilator.

Fig.3

**Description**

Technical Field

The present disclosure relates to the field of coronary imaging, and in particular to, a method for measuring a fractional flow reserve without a vasodilator.

Background

Fractional flow reserve (FFR) may indicate an influence of coronary stenosis lesion on distal blood flow and diagnosis of myocardial ischemia, and has become a recognized index of the functional assessment of the coronary stenosis.
FFR is defined as a ratio of the maximum blood flow to the myocardium in the innervated region provided by stenosed coronary artery to the maximum blood flow to the myocardium provided by the same normal coronary artery. The FFR may be simplified as a ratio of a mean intracoronary pressure (Pd) at a stenosed distal end in the myocardium's maximum hyperemia state to a mean aortic pressure (Pa) at a coronary artery inlet, that is, FFR = Pd/Pa.
Existing methods used for calculating the fractional flow reserve (FFR) include:

Method 1: measuring the pressure (Pd) at the stenosed distal end of the coronary artery in the myocardium's maximum hyperemia state by a pressure guide wire, thereby calculating the FFR;

Method 2: according to the published patent "coronary blood flow reserve fraction calculation method based on X-ray coronary angiography image", acquiring a three-dimensional morphology of blood vessels based on a coronary angiography image, calculating blood flow and calculating the mean intracoronary pressure (Pd) at the stenosed distal end by fluid dynamics stimulation to calculate the FFR.

The FFR in the above two methods is calculated based on a blood flow velocity and the mean aortic pressure (Pa) at the coronary artery inlet in the myocardium's maximum hyperemia state, and the mean intracoronary pressure (Pd) at the stenosed distal end obtained by different technical means. However, intracoronary injection or intravenous injection of adenosine or ATP is required for maximum hyperemia of the myocardium, and the injection of adenosine or ATP will decrease the aortic pressure and will cause a certain side effects, such as atrioventricular block, sinus bradycardia, and sinus arrest, and contraindications include second degree atrioventricular block, third degree atrioventricular block, sinoatrial node diseases, tracheal or bronchial asthma, and adenosine allergy.

Summary

An object of the present disclosure is to provide a method for measuring a fractional flow reserve without a vasodilator, detecting myocardial ischemia for patients suffering from coronary heart disease by conventional coronary angiography surgery, namely calculating the fractional flow reserve by conventional angiography images, an aortic pressure and a blood flow, without using the vasodilator (i.e., without the maximum hyperemia state of the myocardium and in the absence of adenosine or ATP), and namely being named as coronary angiography fractional flow reserve (coronary angiography Fractional Flow Reserve, CAFFR, using the abbreviation in the following).
The technical solution of the present disclosure is that a method for measuring a fractional flow reserve without a vasodilator, characterized in that, comprising:

Step S1: measuring a pressure P1 at a coronary artery inlet of heart by a blood pressure sensor;

Step S2: acquiring a two-dimensional vessel diameter and length of a blood vessel by an angiography image, and generating a three-dimensional blood vessel grid model and acquiring a three-dimensional vessel diameter and length of the blood vessel based on two angiography images at an angle of more than 30°;

Step S3: measuring a time taken for blood flowing from a start point to an end point of a segment of the blood vessel and calculating a blood flow velocity V1 based on the time and three-dimensional length of the blood vessel;

Step S4: calculating an aortic pressure P2 in a maximum hyperemia state;
calculating the aortic pressure P2 according to the following formulas:

$$P2 = P1*0.85, \text{ when } P1 \leq 95\text{mmHg,}$$

and

$$P2 = P1*0.80, \text{ when } P1 > 95\text{mmHg;}$$

Step S5: calculating a blood flow velocity V2 in the maximum hyperemia state:
calculating the blood flow velocity V2 according to the following formulas:

$$V2 = 1.65*V1 + 60, \text{ when } V1 \leq 100\text{mm/s;}$$

$$V2 = 1.23*V1 + 100, \text{ when } 100\text{mm/s} < V1 \leq 200\text{mm/s;}$$

$$V2 = 0.95*V1 + 150, \text{ when } V1 > 200\text{mm/s;}$$

Step S6: taking the blood flow velocity V2 in the maximum hyperemia state calculated in the Step S5 as a velocity at the coronary artery inlet, taking the aortic pressure P2 in the maximum hyperemia state calculated in the Step S4 as a pressure Pa at the coronary artery inlet, calculating a pressure drop $\Delta$P from the coronary artery inlet to a distal end of a coronary stenosis, calculating a mean intracoronary pressure Pd at the distal end of the stenosis based on the formula of Pd = Pa-$\Delta$P, and calculating coronary angiography fractional flow reserve CAFFR based on the formula of CAFFR=Pd/Pa.

As a preferred embodiment, a specific manner for measuring a pressure P1 at a coronary artery inlet of heart in the Step S1 comprises:
connecting a pressure pipe of the blood pressure sensor to a three-way manifold, connecting an angiography catheter to the coronary artery inlet of heart, filling the pressure pipe of the blood pressure sensor with saline, maintaining the blood pressure sensor and the heart at the same horizontal level, and a pressure value measured by the blood pressure sensor being the pressure P1 at the coronary artery inlet of heart.

As a preferred embodiment, a specific manner for generating a three-dimensional blood vessel grid model in the Step S2 comprises:

subjecting two-dimensional structural data to three-dimensional reconstruction and acquiring three-dimensional structural data of the blood vessel segments, and the two-dimensional structural data being from two blood vessel segments which are on two X-ray coronary angiography images at different angles and have a mapping relationship with each other;

repeating the above steps until accomplishing the three-dimensional reconstructions for all of blood vessel segments, and merging reconstructed blood vessel segments for obtaining a complete three-dimensional blood vessel.

As a preferred embodiment, a specific manner for calculating a blood flow velocity V1 in the Step S3 comprises:

acquiring a specified heart rate H times/minute from a patient, acquiring an image frequency S frame/second from angiography image information, and calculating the number of frames X for the images according to the following formula: $X = (1 \div (H \div 60)) \times S$;

according to the number of frames through which the images passes during one cardiac cycle, acquiring a start point of the cardiac cycle from an image corresponding to a two-dimensional starting frame and an end point of the cardiac cycle from an image corresponding to a two-dimensional end frame, respectively; and intercepting a length of the blood vessel within one cardiac cycle from three-dimensional synthetic data by using the start point and the end point;

obtaining the blood flow velocity V1 based on the formula 1: $P = X \div S$ and formula 2: $V1 = L \div P$, wherein a length of the intercepted blood vessel is L and a time taken for one cardiac cycle is P.

As a preferred embodiment, a specific manner for calculating a pressure drop ΔP from the coronary artery inlet to a distal end of a coronary stenosis in the Step S6 comprises:

solving a fundamental formula of incompressible flow based on the blood flow velocity and the three-dimensional blood vessel grid model; solving the three-dimensional blood vessel grid model; and solving a continuity equation and a Navier-Stokes equation by a numerical method,

$$\nabla \cdot \vec{V} = 0 \#$$

$$\rho \frac{\partial \vec{V}}{\partial t} + \rho \vec{V} \cdot \nabla \vec{V} = -\nabla P + \nabla \cdot \mu \left( \nabla \vec{V} + (\nabla \vec{V})^T \right)$$

wherein, $\vec{V}$ is the blood flow velocity, $P$ is the pressure, $\rho$ is a blood density, and $\mu$ is a blood viscosity;

an inlet boundary condition is the blood flow velocity, and an outlet boundary condition is out-flow boundary condition;

calculating the pressures drop ΔP from the inlet to each point downstream along a center line of the blood vessel.

Advantages of the present disclosure are:

1.The present disclosure detects myocardial ischemia for patients suffering from coronary heart disease by conventional coronary angiography surgery, namely calculating the coronary angiography fractional flow reserve (CAFFR) by conventional angiography images, an aortic pressure and a blood flow, without using the vasodilator (i.e., without the maximum hyperemia state of the myocardium and in the absence of adenosine or ATP).

Brief Description of Drawings

The present disclosure will be further described below with reference to the drawings and embodiments

Fig.1 is a comparison distribution diagram showing the measured aortic pressure data in a resting state and aortic pressure data in a maximum hyperemia state;

Fig.2 is a comparison distribution diagram showing the measured blood flow velocity data in a resting state and blood flow velocity data in a maximum hyperemia state.

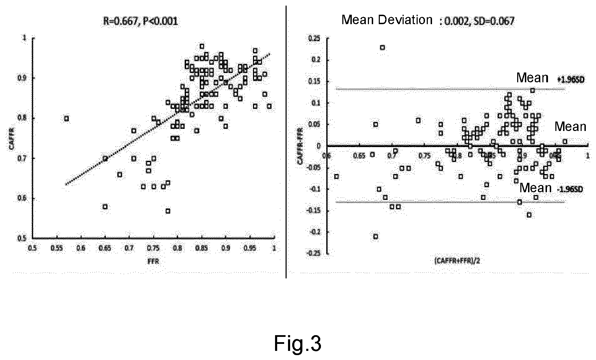

Fig.3 is a schematic comparison diagram showing the relevance and consistency of the CAFFR and FFR.

DEENDED DESCRIPTION OF PREFERRED EMBODIMENTS

Embodiment 1: a method for measuring a fractional flow reserve without a vasodilator, characterized in that, comprises the following steps:

Step S1: measuring a pressure P1 at a coronary artery inlet of heart by a blood pressure sensor, and its specific manner comprising:
connecting a pressure pipe of the blood pressure sensor to a multi-connected three-way manifold, connecting an angiography catheter to the coronary artery inlet of heart, filling the pressure pipe of the blood pressure sensor with saline, maintaining the blood pressure sensor and the heart at the same horizontal level, and a pressure value measured by the blood pressure sensor being the pressure P1 at the coronary artery inlet of heart;

Step S2: acquiring a two-dimensional vessel diameter and length of a blood vessel by an angiography image, and generating a three-dimensional blood vessel grid model and acquiring a three-dimensional vessel diameter and length of the blood vessel based on two angiography images at an angle of more than 30°;

a specific manner for a three-dimensional blood vessel grid model being as follows:

subjecting two-dimensional structural data from two blood vessel segments which are on two X-ray coronary angiography images at different angles and have a mapping relationship with each other to three-dimensional reconstruction, and acquiring three-dimensional structural data of the blood vessel segments;

repeating the above steps until accomplishing the three-dimensional reconstructions for all of blood vessel segments, and merging reconstructed blood vessel segments for obtaining a complete three-dimensional blood vessel;

Step S3: measuring a time taken for blood flowing from a start point to an end point of a segment of the blood vessel and calculating a blood flow velocity V1 based on the time and three-dimensional length of the blood vessel, and its specific manner being as follows:

acquiring a specified heart rate H times/minute from a patient, acquiring an image frequency S frame/second from angiography image information, and calculating the number of frames X for the images according to the following formula: $X = (1 \div (H \div 60)) \times S$;

according to the number of frames through which the images passes during one cardiac cycle, acquiring a start point of the cardiac cycle from an image corresponding to a two-dimensional starting frame and an end point of the cardiac cycle from an image corresponding to a two-dimensional end frame, respectively; and intercepting a length of the blood vessel within one cardiac cycle from three-dimensional synthetic data by using the start point and the end point;

obtaining the blood flow velocity V1 based on the formula 1: $P = X \div S$ and formula 2: $V1 = L \div P$, wherein a length of the intercepted blood vessel is L and a time taken for one cardiac cycle is P;

Step S4: calculating an aortic pressure P2 in a maximum hyperemia state;

calculating the aortic pressure P2 according to the following formulas:

$$P2 = P1 * 0.85,$$

when P1≤95mmHg;

$$P2 = P1 * 0.80,$$

when P1 > 95mmHg.

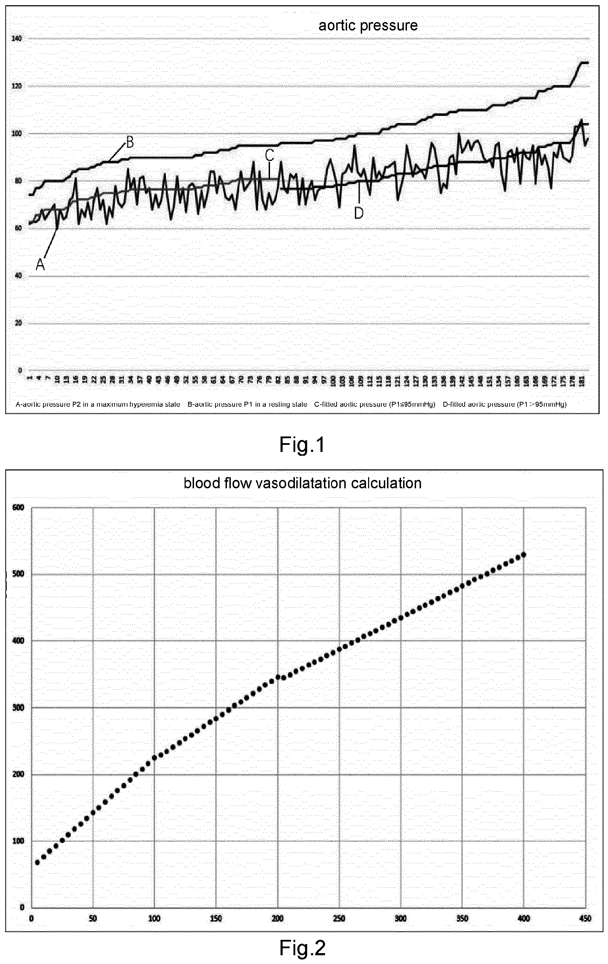

Wherein Fig.1 is a comparison distribution diagram showing the measured aortic pressure data in a resting state and aortic pressure data in a maximum hyperemia state, Line B indicates the aortic pressure in the resting state, Line A indicates the aortic pressure in the maximum hyperemia state, and Lines C and D indicate the fitted aortic pressures in the maximum hyperemia state for each section.

Step S5: calculating a blood flow velocity V2 in the maximum hyperemia state;
calculating the blood flow velocity V2 according to the following formulas:

$$V2 = 1.65 * V1 + 60$$

when V1≤100mm/s;

$$V2=1.23*V1+100$$

when 100mm/s<V1≤200mm/s;

$$V2=0.95*V1+150$$

when V1>200mm/s;

Fig.2 is a comparison distribution diagram showing the measured blood flow velocity data in a resting state and blood flow velocity data in a maximum hyperemia state, wherein the horizontal ordinate indicates a resting blood flow velocity and the longitudinal coordinate indicates a blood flow velocity in the maximum hyperemia state.

Step S6: taking the blood flow velocity V2 in the maximum hyperemia state calculated in the Step S5 as a velocity at the coronary artery inlet, taking the aortic pressure P2 in the maximum hyperemia state calculated in the Step S4 as a pressure Pa at the coronary artery inlet, calculating a pressure drop ∆P from the coronary artery inlet to a distal end of a coronary stenosis, calculating a mean intracoronary pressure Pd at the distal end of the stenosis based on the formula of Pd = Pa-∆P, and calculating coronary angiography fractional flow reserve CAFFR based on the formula of CAFFR=Pd/Pa.

A specific manner for calculating a pressure drop ∆P from the coronary artery inlet to a distal end of a coronary stenosis in the Step S6 comprises:

solving a fundamental formula of incompressible flow based on the blood flow velocity and the three-dimensional blood vessel grid model; solving the three-dimensional blood vessel grid model; and solving a continuity equation and a Navier-Stokes equation by a numerical method:

$$\nabla \cdot \vec{V} = 0_{\#}$$

$$\rho \frac{\partial \vec{v}}{\partial t} + \rho \vec{V} \cdot \nabla \vec{V} = -\nabla P + \nabla \cdot \mu(\nabla \vec{V} + (\nabla \vec{V})^{T})$$

wherein, $\vec{V}$ is the blood flow velocity, $P$ is the pressure, $\rho$ is a blood density, and $\mu$ is a blood viscosity;

an inlet boundary condition is the blood flow velocity, and an outlet boundary condition is out-flow boundary condition;

calculating the pressures drop ∆P from the inlet to each point downstream along a center line of the blood vessel.

The calculation results of the coronary angiography fractional flow reserves CAFFRs in Embodiment 1 are shown in Table 1.

Meanwhile, Table 1 also shows comparisons between the calculated CAFFRs and the calculated FFRs.

| | the calculated CAFFRs≤0.8 | the calculated CAFFRs>0.8 |
|---|---|---|
| FFRs≤0.8, the pressure guide wire | 21 | 4 |
| FFRs>0.8, the pressure guide wire | 4 | 91 |
| | Measured values (%) | Number of patients |
| accuracy | 93 | 120 |
| sensitivity | 84 | 25 |
| specificity | 96 | 95 |
| positive predictive value | 84 | 25 |
| negative predictive value | 96 | 95 |

It can be concluded from Fig.1 and Table 1 that calculation result accuracy of the coronary angiography fractional flow reserve CAFFR is more than 90% and the CAFFRs and the FFRs have excellent relevance and consistency.

The embodiments mentioned above are merely intended to be illustrative of the principles and effectives of the present disclosure and not to be limitations to the present disclosure. A person skilled in the art can make various changes or modifications to be above embodiments without departing from the spirit or scope thereof. Therefore, all equivalent modifications or changes made by those with ordinary knowledge in the technical field without departing from the spirit and technical idea disclosed by the present disclosure should be considered as being covered by the claims thereof.

**Claims**

1. A method for measuring a fractional flow reserve without a vasodilator, **characterized in that**, comprising:

   Step S1: measuring a pressure P1 at a coronary artery inlet of heart by a blood pressure sensor;
   Step S2: acquiring a two-dimensional vessel diameter and length of a blood vessel by an angiography image, and generating a three-dimensional blood vessel grid model and acquiring a three-dimensional vessel diameter and length of the blood vessel based on two angiography images at an angle of more than 30°;
   Step S3: measuring a time taken for blood flowing from a start point to an end point of a segment of the blood vessel and calculating a blood flow velocity V1 based on the time and three-dimensional length of the blood vessel;
   Step S4: calculating an aortic pressure P2 in a maximum hyperemia state;
   calculating the aortic pressure P2 according to the following formulas:

   $$P2=P1*0.85, \text{ when } P1 \leq 95mmHg;$$

   $$P2=P1*0.80, \text{ when } P1 > 95mmHg;$$

   Step S5: calculating a blood flow velocity V2 in the maximum hyperemia state;
   calculating the blood flow velocity V2 according to the following formulas:

   $$V2=1.65*V1+60, \text{ when } V1 \leq 100mm/s;$$

   $$V2=1.23*V1+100, \text{ when } 100mm/s < V1 \leq 200mm/s;$$

   $$V2=0.95*V1+150, \text{ when } V1 > 200mm/s;$$

   Step S6: taking the blood flow velocity V2 in the maximum hyperemia state calculated in the Step S5 as a velocity at the coronary artery inlet, taking the aortic pressure P2 in the maximum hyperemia state calculated in the Step S4 as a pressure Pa at the coronary artery inlet, calculating a pressure drop $\Delta P$ from the coronary artery inlet to a distal end of a coronary stenosis, calculating a mean intracoronary pressure Pd at the distal end of the stenosis based on the formula of Pd = Pa-$\Delta$P, and calculating coronary angiography fractional flow reserve CAFFR based on the formula of CAFFR=Pd/Pa.

2. The method for measuring a fractional flow reserve without a vasodilator according to claim 1, **characterized in that**, a specific manner for measuring a pressure P1 at a coronary artery inlet of heart in the Step S1 comprises: connecting a pressure pipe of the blood pressure sensor to a three-way manifold, connecting an angiography catheter to the coronary artery inlet of heart, filling the pressure pipe of the blood pressure sensor with saline, maintaining the blood pressure sensor and the heart at the same horizontal level, and a pressure value measured by the blood pressure sensor being the pressure P1 at the coronary artery inlet of heart.

3. The method for measuring a fractional flow reserve without a vasodilator according to claim 1, **characterized in that**, a specific manner for generating a three-dimensional blood vessel grid model in the Step S2 comprises:

subjecting two-dimensional structural data to three-dimensional reconstruction and acquiring three-dimensional structural data of the blood vessel segments, and the two-dimensional structural data being from two blood vessel segments which are on two X-ray coronary angiography images at different angles and have a mapping relationship with each other;

repeating the above steps until accomplishing the three-dimensional reconstructions for all of blood vessel segments, and merging reconstructed blood vessel segments for obtaining a complete three-dimensional blood vessel.

**4.** The method for measuring a fractional flow reserve without a vasodilator according to claim 1, **characterized in that**, a specific manner for calculating a blood flow velocity V1 in the Step S3 comprises:

acquiring a specified heart rate H times/minute from a patient, acquiring an image frequency S frame/second from angiography image information, and calculating the number of frames X for the images according to the following formula: $X = (1 \div (H \div 60)) \times S$;

according to the number of frames through which the images passes during one cardiac cycle, acquiring a start point of the cardiac cycle from an image corresponding to a two-dimensional starting frame and an end point of the cardiac cycle from an image corresponding to a two-dimensional end frame, respectively; and intercepting a length of the blood vessel within one cardiac cycle from three-dimensional synthetic data by using the start point and the end point; and

obtaining the blood flow velocity V1 based on the formula 1: $P = X \div S$ and formula 2: $V1 = L \div P$, wherein a length of the intercepted blood vessel is L and a time taken for one cardiac cycle is P.

**5.** The method for measuring a fractional flow reserve without a vasodilator according to claim 1, **characterized in that**, a specific manner for calculating a pressure drop ΔP from the coronary artery inlet to a distal end of a coronary stenosis in the Step S6 comprises:

solving a fundamental formula of incompressible flow based on the blood flow velocity and the three-dimensional blood vessel grid model; solving the three-dimensional blood vessel grid model; and solving a continuity equation and a Navier-Stokes equation by a numerical method,

$$\nabla \cdot \vec{V} = 0_{\#}$$

$$\rho \frac{\partial \vec{V}}{\partial t} + \rho \vec{V} \cdot \nabla \vec{V} = -\nabla P + \nabla \cdot \mu (\nabla \vec{V} + (\nabla \vec{V})^T)$$

wherein, $\vec{V}$ is the blood flow velocity, $P$ is the pressure, $\rho$ is a blood density, and $\mu$ is a blood viscosity;

an inlet boundary condition is the blood flow velocity, and an outlet boundary condition is out-flow boundary condition;

calculating the pressures drop ΔP from the inlet to each point downstream along a center line of the blood vessel.

**Fig.1**

**Fig.2**

Fig.3

## INTERNATIONAL SEARCH REPORT

| International application No. |
| --- |
| **PCT/CN2018/091166** |

| | |
| --- | --- |
| **A.    CLASSIFICATION OF SUBJECT MATTER** |
| A61B 5/026(2006.01)i;  A61B 6/00(2006.01)i |
| According to International Patent Classification (IPC) or to both national classification and IPC |

| | |
| --- | --- |
| **B.    FIELDS SEARCHED** |
| Minimum documentation searched (classification system followed by classification symbols) |
| A61B 5/-; A61B 6/- |
| Documentation searched other than minimum documentation to the extent that such documents are included in the fields searched |
| Electronic data base consulted during the international search (name of data base and, where practicable, search terms used) |
| CNKI, CNPAT, WPI, EPODOC: 霍云飞, 苏州润迈德医疗科技, 血流储备分数, 动脉, 心脏, 冠状, 造影, 血管, 夹角, 血压, 最大, 充血, FFR, fraction, flow, reserve, coronary, cardio, heart, Max, hyperemia, expand, expansion, dilate, ATP |

**C.    DOCUMENTS CONSIDERED TO BE RELEVANT**

| Category* | Citation of document, with indication, where appropriate, of the relevant passages | Relevant to claim No. |
| --- | --- | --- |
| A | CN 105138813 A (SIEMENS AG) 09 December 2015 (2015-12-09) description, paragraphs [0037]-[0049], and figure 4 | 1-5 |
| A | CN 106327487 A (SUZHOU RUNXIN MEDICAL TECHNOLOGY CO., LTD.) 11 January 2017 (2017-01-11) entire document | 1-5 |
| A | CN 105764411 A (MEDTRONIC VASCULAR GALWAY LIMITED) 13 July 2016 (2016-07-13) entire document | 1-5 |
| A | US 2003032886 A1 (DGANY, E. ET AL.) 13 February 2003 (2003-02-13) entire document | 1-5 |
| A | CN 105190630 A (CATHWORKS LTD.) 23 December 2015 (2015-12-23) entire document | 1-5 |
| A | CN 105326486 A (SHANGHAI JIAOTONG UNIVERSITY ET AL.) 17 February 2016 (2016-02-17) entire document | 1-5 |

| | |
| --- | --- |
| ☐ Further documents are listed in the continuation of Box C. | ☑ See patent family annex. |

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **05 November 2018** | **26 November 2018** |

| Name and mailing address of the ISA/CN | Authorized officer |
| --- | --- |
| **State Intellectual Property Office of the P. R. China (ISA/ CN)** **No. 6, Xitucheng Road, Jimenqiao Haidian District, Beijing 100088 China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.

**PCT/CN2018/091166**

| Patent document cited in search report | | | Publication date (day/month/year) | Patent family member(s) | | | Publication date (day/month/year) |
|---|---|---|---|---|---|---|---|
| CN | 105138813 | A | 09 December 2015 | US | 2015348260 | A1 | 03 December 2015 |
|    |            |   |                  | EP | 2949268    | A1 | 02 December 2015 |
| CN | 106327487 | A | 11 January 2017 | CN | 106327487 | B | 02 January 2018 |
| CN | 105764411 | A | 13 July 2016 | EP | 3068292 | A1 | 21 September 2016 |
|    |           |   |              | WO | 2015073427 | A1 | 21 May 2015 |
|    |           |   |              | US | 9877660 | B2 | 30 January 2018 |
|    |           |   |              | US | 2015133799 | A1 | 14 May 2015 |
| US | 2003032886 | A1 | 13 February 2003 | AU | 3187900 | A | 28 September 2000 |
|    |            |    |                  | EP | 1251769 | A1 | 30 October 2002 |
|    |            |    |                  | JP | 2003525067 | A | 26 August 2003 |
|    |            |    |                  | US | 6471656 | B1 | 29 October 2002 |
|    |            |    |                  | WO | 0053081 | A1 | 14 September 2000 |
| CN | 105190630 | A | 23 December 2015 | WO | 2014111929 | A1 | 24 July 2014 |
|    |           |   |                  | EP | 2946319 | A1 | 25 November 2015 |
|    |           |   |                  | JP | 2016511649 | A | 21 April 2016 |
|    |           |   |                  | EP | 2946321 | A1 | 25 November 2015 |
|    |           |   |                  | IL | 239961 | D0 | 31 August 2015 |
|    |           |   |                  | IL | 239960 | D0 | 31 August 2015 |
|    |           |   |                  | US | 2014200867 | A1 | 17 July 2014 |
|    |           |   |                  | US | 2015335304 | A1 | 26 November 2015 |
|    |           |   |                  | US | 9977869 | B2 | 22 May 2018 |
|    |           |   |                  | JP | 2016509501 | A | 31 March 2016 |
|    |           |   |                  | WO | 2014111929 | A9 | 28 August 2014 |
|    |           |   |                  | WO | 2014111930 | A1 | 24 July 2014 |
|    |           |   |                  | US | 9858387 | B2 | 02 January 2018 |
|    |           |   |                  | WO | 2014111927 | A1 | 24 July 2014 |
|    |           |   |                  | US | 2017364658 | A1 | 21 December 2017 |
|    |           |   |                  | KR | 20150110609 | A | 02 October 2015 |
|    |           |   |                  | US | 2018268941 | A1 | 20 September 2018 |
|    |           |   |                  | IL | 239960 | A | 31 August 2015 |
|    |           |   |                  | IL | 239961 | A | 31 August 2015 |
|    |           |   |                  | US | 2015339847 | A1 | 26 November 2015 |
|    |           |   |                  | US | 2015342551 | A1 | 03 December 2015 |
|    |           |   |                  | US | 9814433 | B2 | 14 November 2017 |
| CN | 105326486 | A | 17 February 2016 | WO | 2017097073 | A1 | 15 June 2017 |
|    |           |   |                  | CN | 105326486 | B | 25 August 2017 |

Form PCT/ISA/210 (patent family annex) (January 2015)